# EUROPEAN PATENT APPLICATION

(11) **EP 4 740 997 A1**
(43) Date of publication of application: **13.05.2026**
(21) Application number: 24211111.0
(22) Date of filing: 06.11.2024
(51) Int. Cl.: A61N 1/375

(54) **IS-1 SPRING SLEEVE WITH BUILT-IN FASTENERS**

(71) Applicant: BIOTRONIK SE & Co. KG, 12359 Berlin (DE)
(72) Inventor: Binias, Sofia, 10587 Berlin (DE); Große, Ines, 14612 Falkensee (DE)
(74) Representative: Biotronik Corporate Services SE

(57) **Abstract**

The present invention relates to a header (11) of an implantable medical device (1), comprising a receptacle (12) configured to receive a plug of an electrode lead and at least one electrical contact (13) arranged in the receptacle (12) and configured to receive and to electrically contact the plug of the electrode lead; the electrical contact (13) comprising: a sleeve (2) comprising an inner side (20) that surrounds an opening (21) of the sleeve (2), and a spring element (3) arranged in the opening (21), wherein the spring element (3) is configured to receive a plug to establish an electrical connection between the spring element (3) and the plug, wherein the spring element (3) is positioned in the opening (21) of the sleeve (2) and secured therein by a mechanical retention.

## Description

The present invention relates to an implant, particularly an active implant such as an implantable cardiac pacemaker or an implantable cardioverter defibrillator. Typically, such an implant requires the possibility of connecting an electrode lead to the implant via a header of the implant. To this end, the header can comprise a receptacle for receiving a connector (e.g. plug) of the electrode lead. Such a plug is usually received in a sleeve comprising a spring element therein that engages with the plug and realizes an electrically conducting connection between the plug and the sleeve.

Typically, the connector complies with a connector standard, e.g., IS-1/DF-2, or IS-4/DF-4, which is typically used in the design of implantable medical devices, particularly for implantable cardiac pacemakers and cardioverter-defibrillators. It is known in the prior art to connect the spring element to the sleeve by way of resistance welding.

However, the process of resistance welding of the spring element into the sleeve results in additional work in the form of providing, maintaining and monitoring a welding system. The welded connection must also be visually inspected. Incorrectly welded parts are either reworked or scrapped. Thus, there is a desire to provide such a connector in a more efficient manner and with reduced costs while ensuring that the spring element is safely fixed to the sleeve.

This problem is solved by a header for an implantable medical device having the features of claim 1. Preferred embodiments of this aspect of the present invention are stated in the corresponding dependent claims and are described below.

According to claim 1, a header of an implantable medical device is disclosed, comprising a receptacle configured to receive a plug of an electrode lead and at least one electrical contact arranged in the receptacle and configured to receive and to electrically contact the plug of the electrode lead, wherein the electrical contact comprises:
- a sleeve comprising an inner side that delimits an opening, particularly a throughopening, of the sleeve, and
- a spring element arranged in said opening, wherein the spring element is configured to receive a plug to establish an electrical connection between the spring element and the plug.

According to the present invention it is particularly envisioned that the spring element is positioned within the opening of the sleeve and fixed therein by a non-permanent mechanical retention, e.g. such as friction or clamping.

Advantageously, as the retention is mechanical, e.g. based on friction or clamping, there is no need for an additional welding connection between the spring element and the sleeve. Particularly, the invention allows to position the spring element in said opening, which may require a non-permanent (e.g. elastic) deformation of the spring element, which then allows to hold the spring element in said opening of the sleeve by a mechanical engagement between the spring element and the sleeve which may be based on friction, clamping or another mechanical interaction between the spring element and the sleeve that is not based on a material connection or an (e.g. adhesive) bond. As there is no welding seam or bond required between the spring element and the sleeve the mechanical retention can be considered as non-permanent as the spring element can in principle be disengaged from the sleeve in a non-destructive fashion.

Thus, the resistance welding process that was previously necessary may be omitted so that the disadvantages relating to the welding process are alleviated. Particularly, the present invention does not require any welding connection between the spring element and the sleeve or another material bond.

According to a preferred embodiment of the present invention, the sleeve comprises or is made out of a material selected from the group comprising: titanium, stainless steel, MP35N, or platinum/iridium, particularly platinum/iridium in a ratio of 90 to10.

Particularly, the sleeve may be formed by additive manufacturing (3D printing) such as laser sintering, or by way of metal injection molding, metal cutting manufacturing, or milling. This allows one to provide the sleeve with a structure facilitating a mechanical interaction with a corresponding structure of the spring element.

Furthermore, according to a preferred embodiment of the present invention, the spring element comprises or is made out of a material selected from the group comprising titanium, stainless steel, MP35N, or platinum/iridium, particularly platinum/iridium in a ratio of 90 to 10.

Further, according to a preferred embodiment of the present invention, for achieving said mechanical retention, the sleeve comprises a protrusion protruding from said inner side of the sleeve, wherein the protrusion is configured to mechanically interact with the spring element to retain the spring element within the opening.

In a preferred embodiment, the sleeve comprises a plurality of protrusions protruding from said inner side of the sleeve, wherein the respective protrusion engages with the spring element to retain the spring element within the opening.

Furthermore, according to a preferred embodiment of the present invention, the protrusion is elongated and particularly extends in an axial direction of the sleeve, along which axial direction said opening of the sleeve extends through the sleeve.

According to yet another preferred embodiment of the present invention, the protrusion is configured to be received in an aperture of the spring element to retain the spring element within the opening of the sleeve.

According to a further preferred embodiment, the sleeve comprises a plurality of protrusions, wherein particularly the respective protrusion extends in said axial direction of the sleeve, and wherein particularly the respective protrusion is received in an associated aperture of the spring element. Particularly, the sleeve comprises three elongated protrusions (e.g. extending in the axial direction, respectively), wherein the elongated protrusions are preferably equidistantly spaced apart along the inner side in a circumferential direction of the sleeve, with each elongated protrusion being received in an associated aperture of the spring element.

According to yet another preferred embodiment of the present invention, the inner side of the sleeve comprises a circumferential first end and an opposing circumferential second end, wherein the first and the second end are opposite one another in said axial direction of the sleeve. Preferably, the protrusion is located on said circumferential first end of the inner side of the sleeve.

Furthermore, in a preferred embodiment of the present invention, the protrusion is flush with a first face side of the sleeve, which first face side delimits and extends around the opening of the sleeve (particularly at the circumferential first end of the inner side sleeve).

Particularly, for engaging / mechanically interacting with the sleeve, the spring element comprises a first end (e.g. in the axial direction) that is configured to butt against the protrusion (e.g. in the axial direction) to retain the spring element within the opening.

In a preferred embodiment of the present invention, the protrusion is an annular protrusion extending along a circumferential direction of the sleeve around the opening.

According to an alternative embodiment of the present invention, the sleeve comprises a plurality of protrusions located on the first end of the inner side of the sleeve.

In a preferred embodiment, the respective protrusion of said plurality of protrusions can be flush with a first face side of the sleeve, which first face side delimits and extends around the opening of the sleeve (particularly at the circumferential first end of the inner side sleeve).

Further, in a preferred embodiment of the present invention, the first end of the spring element is configured to butt against the respective protrusion (particularly in the axial direction) to retain the spring element within the opening.

Furthermore, in a preferred embodiment, the sleeve comprises three protrusions arranged on the first end of the inner side, wherein the protrusions are preferably equidistantly spaced apart along the first end of the inner side in a circumferential direction of the sleeve.

According to yet another preferred embodiment of the present invention, for achieving said mechanical retention of the spring element. The sleeve comprises a further protrusion protruding from said inner side of the sleeve, wherein the further protrusion is configured to mechanically interact with the spring element to retain the spring element within the opening.

In a preferred embodiment of the present invention, the further protrusion is located on the second end of the inner side of the sleeve.

Furthermore, in a preferred embodiment of the present invention, the further protrusion is flush with a second face side of the sleeve, which second face side delimits and extends around the opening of the sleeve (at the second end of the inner side of the sleeve) and faces away from the first face side of the sleeve.

According to yet another preferred embodiment of the present invention, the spring element comprises a second end (e.g. in the axial direction, particularly opposite the first end of the spring element) configured to butt against the further protrusion (e.g. in the axial direction) to retain the spring element within the opening,

Furthermore, in the preferred embodiment of the present invention, the further protrusion is an annular protrusion extending along the circumferential direction of the sleeve around the opening of the sleeve.

Further, according to a preferred alternative embodiment of the present invention, the sleeve comprises a plurality of further protrusions located on the second end of the inner side. In a preferred embodiment, the respective further protrusion can be flush with a second face side of the sleeve, which second face side delimits and extends around the opening of the sleeve (particularly at the second end of the inner side of the sleeve).

Further, in a preferred embodiment, the second end of the spring element is configured to butt against the respective further protrusion (e.g. in the axial direction) to retain the spring element within the opening of the sleeve.

Furthermore, in a preferred embodiment, the sleeve comprises three further protrusions arranged on the second end of the inner side, wherein the further protrusions are preferably equidistantly spaced apart along the second end of the inner side in the circumferential direction of the sleeve.

According to yet another preferred embodiment of the present invention, the spring element is a bent metal plate and comprises two opposing end portions, namely a first end portion and an opposing second end portion, the respective end portion forming an open ring, and wherein the two end portions are connected (particularly integrally connected) by axial struts being spaced apart in a circumferential direction of the spring element.

Particularly, in a preferred embodiment, the first end portion forms said first end of the sleeve and the second end portion forms said second end of the sleeve (see above).

According to a further aspect, an implantable medical device is provided, wherein the implantable medical device comprises a header according to the invention. In one embodiment, the implantable medical device is a cardiac pacemaker, a cardioverter/defibrillator or a neurostimulator.

In one embodiment, the header is arranged on a housing of the implantable medical device. In one embodiment, the housing is made from an electrically conductive, biocompatible material, e.g., stainless steel, titanium or a titanium alloy, or a biocompatible polymer, e.g., a liquid crystal polymer, or a conductive polymer. In some embodiments, the implantable medical device comprises an electrical feedthrough comprising an electrical insulator, particularly made form a ceramic, e.g., alumina (Al₂O₃), a glass, a glass solder, or plastic material, e.g., a synthetic resin (e.g., an epoxy resin) or a thermoplastic material (e.g., a liquid crystal polymer), at least one feedthrough conductor, e.g., a feedthrough pin made from, e.g., platinum, iridium, platinum/iridium, niobium, titanium, molybdenum, etc., the feedthrough conductor extending through the electrical insulator, and optionally a flange or ferrule surrounding the electrical insulator. In one embodiment, the implantable medical device comprises an operational circuit or electronic module configured to perform the intended function of the medical device, e.g., cardiac sensing and/or cardiac pacing, neurostimulation, etc. In one embodiment, the operational circuit or electronics module is in electrical connection with the electric contact in the header via the electrical feedthrough, wherein particularly the electrical contact is electrically connected, and particularly fixed to, e.g., welded, to the feedthrough conductor via an electrical conductor.

In the following, embodiments of the present invention as well as further features and advantages of embodiments of the present invention shall be described with reference to the Figures, wherein
- Fig. 1: shows an illustration of an embodiment of a medical implant according to the present invention with a header having a receptacle and an electrical contact arranged in the receptacle, the electrical contact comprising a spring element positioned in an opening of a corresponding sleeve and secured therein by a mechanical retention,
- Figs. 2A-B: show an embodiment of a spring element (A) as it can be used in the implant shown in Fig. 1, and a blank (B) from which the spring element can be made, particularly by bending,
- Figs. 3A-D: show an embodiment of the present invention, wherein the spring element is retained by elongated (e.g. axial) protrusions of the sleeve,
- Figs. 4A-D: show an embodiment of the present invention, wherein the spring element is retained by annular protrusions of the sleeve, and
- Figs. 5A-D: show an embodiment of the present invention, wherein the spring element is retained by a plurality of protrusions arranged at each of the two opposing ends of the spring element.

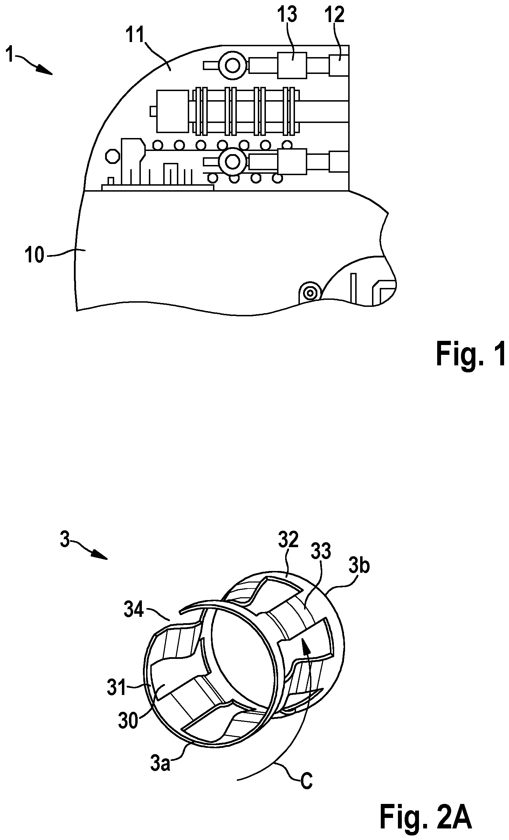

Fig. 1 shows an embodiment of an implant 1 according to the present invention. Particularly, the implant 1 is an active implant such as an implantable cardiac pacemaker, an implantable cardioverter defibrillator, an implantable neurostimulator, or another active implant. Particularly, such an implant 1 typically requires the possibility of connecting an electrode lead to the implant 1 via a header 11 of the implant 1. To this end, the header 11 can comprise a receptacle 12 for receiving a connector (e.g. plug) of the electrode lead. The implant 1, particularly said receptacle 12, further accommodates an electrical contact 13. The electrical contact comprises a sleeve 2 having an inner side 20 that delimits an opening 21 of the sleeve 2. A spring element 3 is arranged in the opening 21 of the sleeve 2, wherein the spring element 3 is configured to receive said plug to establish an electrical connection between the spring element 3 and the plug. Furthermore, an electrical connection between the spring element 3 and the sleeve 2 can be achieved due to the spring element 3 contacting the sleeve 2. Furthermore, an electrical connection between the spring element 3 / plug and an electronic component of the implant 1 (e.g. an electronic module accommodated in a housing 10 of the implant 1) may be made via the sleeve 2, e.g. by means of a wiring connected to the sleeve 2 in an electrically conducting fashion..

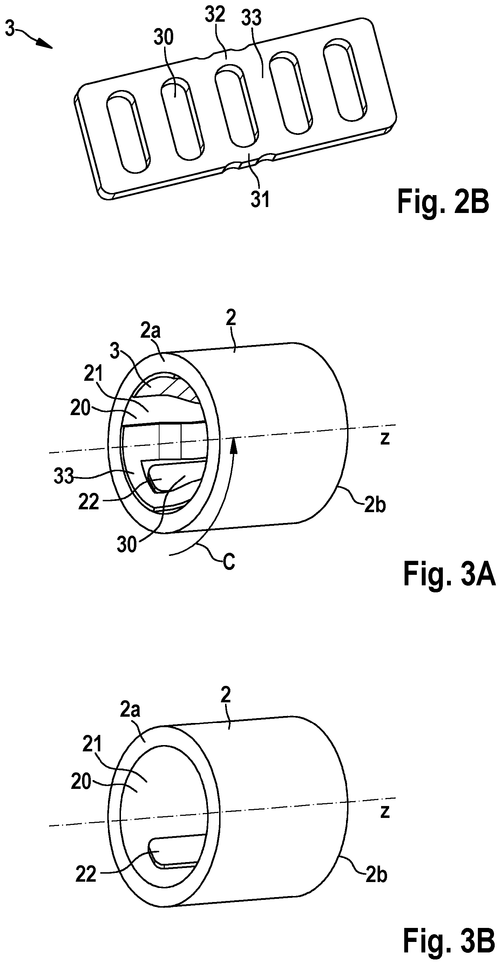

Fig. 3A to 3D show a first embodiment of the present invention. Generally, in all embodiments described herein, the sleeve 3 may be formed as a hollow cylindrical body defining an opening 21 therein that extends through the sleeve 2 in an axial direction z.

Furthermore, the sleeve 2 comprises an inner side 20 that delimits said opening 21 of the sleeve 2.

Particularly, the spring element 3 may comprise a tubular structure that may be formed from a bent metal plate as indicated in Figs. 2A-2B. The spring element 3 may comprise a first and a second end portion 31, 32 arranged opposite one another in the axial direction z, wherein the respective end portion 31, 32 may each form an open ring. Particularly, the two end portions 31, 32 may be connected (particularly integrally connected) by axial struts 33 that are spaced apart in a circumferential direction C1 of the spring element 3 and each extend along the axial direction z. Between said struts 33, apertures 30 are formed, that may be used for engaging with the structure provided on the inner side 20 of the sleeve 2. Due to the open ring structure of said end portions 31, 32, the spring element 3 comprises a continuous axial gap 34 (cf. Fig. 2A). This allows one to compress the spring element 3 (against a restoring force generated by the spring element 3) in the radial direction such that the gap 34 and at the same time an outer diameter of the spring element 3 are reduced. As will be described in the following, this may be used to insert the spring element 3 into said opening 21 of the sleeve 2 to facilitate engagement of the spring element 3 with said structures provided on the inner side 20 of the sleeve 2 upon expansion of the spring element 3 towards its initial uncompressed state.

Furthermore, the axial struts 33 may comprise a curvature so that the spring element 3 forms a constriction that allows an intimate contact between the struts 33 of the spring element 3 and a plug that tensions the struts 33 when being plugged into the opening 21 and constriction formed by the spring element 3.

Particularly, according to an embodiment of the invention shown in Fig. 3A to 3D, the sleeve 2 may comprise at least one protrusion 22 protruding from said inner side 20 of the sleeve 2, wherein the protrusion 22 is configured to engage with the spring element 3 to retain the spring element 3 in the opening 21. To this end, the protrusion 22 can engage with an aperture of the spring element 3 which can be one of the apertures 30 described above.

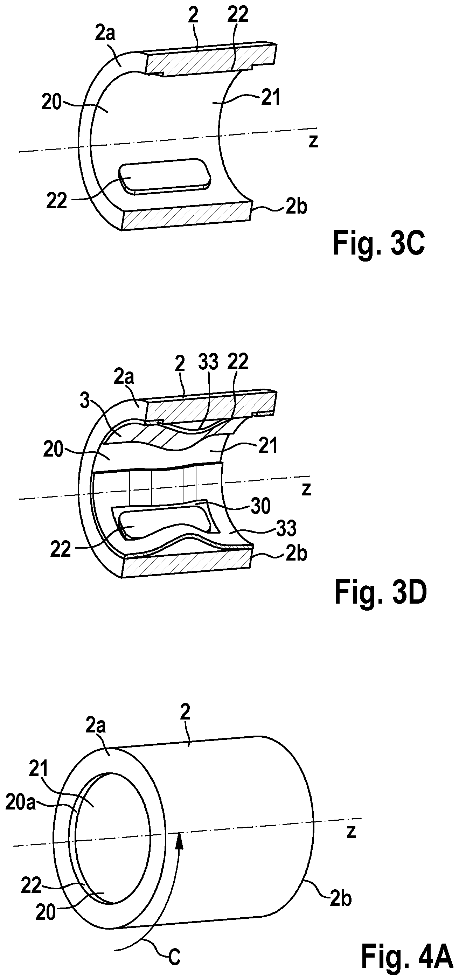

As shown in Fig. 3C, the protrusion 22 is an elongated protrusion 22 that extends in the axial direction z of the sleeve 3 and may be configured to engage with said aperture 30 of the spring element 3 in a form-fitting manner as indicated in Fig. 3D.

In a preferred embodiment, the sleeve 2 comprises a plurality of protrusions 22 protruding from said inner side 20 of the sleeve 2, wherein the respective protrusion 22 engages with the spring element 3 to retain the spring element 3 in the opening 21. Particularly, the sleeve 2 may comprise several such elongated protrusions 22 which may be spaced apart from one another in the circumferential direction C of the sleeve 2, wherein each elongated protrusion 22 may engage with an associated aperture 30 of the spring element 3.

Due to the protrusion (2) 22, the spring element 3 is retained in the opening 21 of the sleeve 2 as the respective protrusion 22 prevents an axial movement of the spring element 3 in the axial direction z. This is due to the fact that the respective protrusion 22 butts against the first end portion 31 as well as against the opposing second end portion 32 of the spring element 3 when engaging into the associated aperture 30 of the spring element 3 (cf. Fig. 3D). In order to overcome this engagement, the spring element 3 would need to be compressed in the radial direction allowing disengagement from the protrusion(s) 22 and removal from the sleeve 2 in the axial direction z.

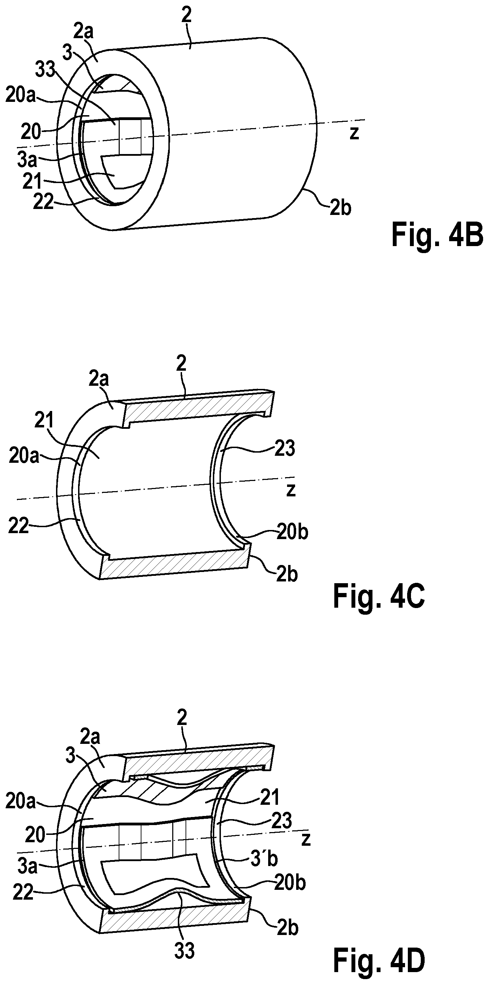

Furthermore, Figs. 4A to 4B show a further preferred embodiment of the present invention. Here, the sleeve 2 comprises an annular protrusion 22 located on a circumferential first end 20a of the inner side 20 of the sleeve 2 and preferably an opposing further annular protrusion 23 located on an opposing circumferential second end 20b of the inner side 20 of the sleeve 2 as e.g. indicated in Fig. 4C. Particularly, as shown in Fig. 4C, the protrusions 22, 23 can be flush with a corresponding face side 2a, 2b of the sleeve 2.

Here, once the spring element 3 has been inserted (e.g. in a compressed state) into the opening 21 of the sleeve 2 as shown e.g. in Fig. 4B, it can re-expand and its opposing ends 3a, 3b formed e.g. by the afore-described first and the second end portion 31, 32 (cf. Fig. 2A) can engage with the annular protrusions 22, 23 by butting against them in the axial direction z (cf. Fig. 4D).

This prevents movement of the spring element 3 in the axial direction z within the opening 21 of the sleeve 2 and thus retains the spring element 3 in the opening 21 of the sleeve 2. Again, the spring element 3 may be removed from the sleeve 2 in the axial direction z by compressing it in the radial direction thus achieving disengagement from the opposing annular protrusions 22, 23 by reducing the outer diameter of the spring element 3.

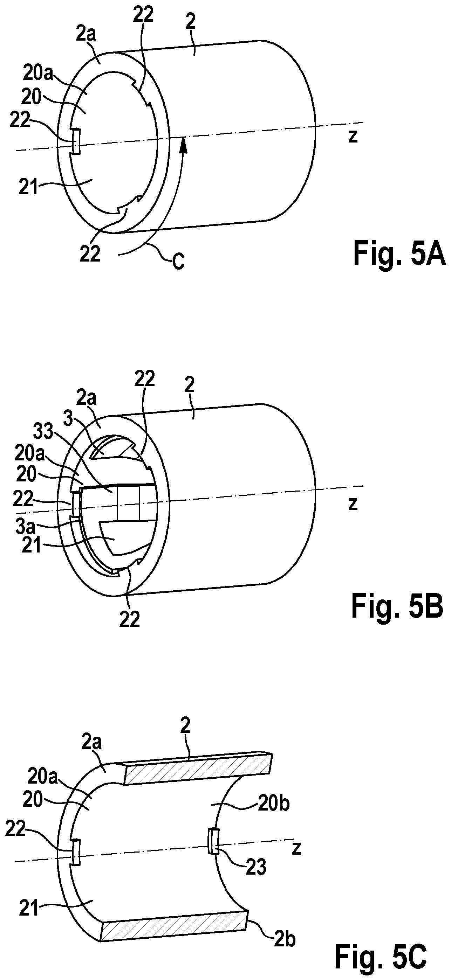

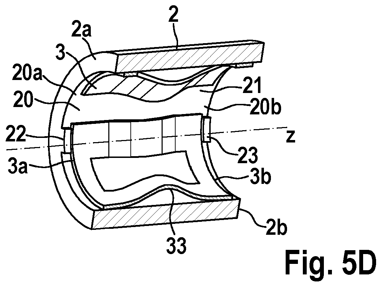

Furthermore, Fig. 5A-5D show a further preferred embodiment of the present invention. Particularly, it is not mandatory that the protrusions 22, 23 shown in Figs. 4A-4B are annular, i.e., extend all the way around the opening 21 of the sleeve 2. Alternatively, the respective protrusion 22, 23 may be a local protrusion 22, 23 as shown in Fig. 5C arranged on the respective end 20a, 20b of the inner side 20. Particularly, the sleeve 2 may comprise several such protrusions 22, 23 on each end 20a, 20b of the inner side 20 as indicated in Fig. 5A. These protrusions 22, 23 may be spaced apart from one another in the circumferential direction C such that they are separated by gaps, but are capable of retaining the spring element 3 in the same fashion as the annular protrusions 22, 23 described in conjunction with Figs. 4A-4D as may be inferred from Fig. 5D. Also, here, the protrusions 22, 23 can be flush with the corresponding face side 2a, 2b of the sleeve 2.

The present invention offers the advantage that an additional welding process is not needed to fix the spring element to its sleeve. This simplifies the production of the corresponding connector and reduces the eject rate.

## Claims

1. Header (11) for an implantable medical device (1), comprising a receptacle (12) configured to receive a plug of an electrode lead and at least one electrical contact (13) arranged in the receptacle (12) and configured to receive and to electrically contact the plug of the electrode lead; the electrical contact (13) comprising:
- a sleeve (2) comprising an inner side (20) that surrounds an opening (21) of the sleeve (2), and
- a spring element (3) arranged in the opening (21), wherein the spring element (3) is configured to receive a plug to establish an electrical connection between the spring element (3) and the plug,
**characterized in that**
the spring element (3) is positioned in the opening (21) of the sleeve (2) and secured therein by a mechanical retention.

2. The header according to claim 1, wherein the sleeve (2) comprises a protrusion (22) protruding from said inner side (20) of the sleeve, wherein the protrusion (22) is configured to engage with the spring element (3) to retain the spring element (3) in the opening (21).

3. The header according to claim 2, wherein the protrusion (22) is elongated, particularly in an axial direction (z) of the sleeve (2).

4. The header according to claim 2 or 3, wherein the protrusion (22) is received in an aperture (30) of the spring element (3) to retain the spring element (3) in the opening (21) of the sleeve (3).

5. The header according to one of the preceding claims, wherein the inner side (20) of the sleeve (2) comprises a circumferential first end (20a) and an opposing circumferential second end (20b).

6. The header according to claims 2 and 5, wherein the protrusion (22) is located on the first end (20a).

7. The header according to claim 2 or according to one of the claims 5 to 6 insofar referring to claim 2, wherein the spring element (3) comprises a first end (3a) configured to butt against the protrusion (22) to retain the spring element (3) in the opening (21).

8. The header according to claim 2 or according to one of claims 3 to 7 insofar referring to claim 2, wherein the protrusion (22) is an annular protrusion.

9. The header according to one of the claims 6 to 8, wherein the sleeve (2) comprises a plurality of protrusions (22) located on the first end (21a) of the inner side (20).

10. The header according to claim 2 or according to one of the claims 3 to 9 insofar referring to claim 2, wherein the sleeve (2) comprises a further protrusion (23) protruding from said inner side (20) of the sleeve (2), wherein the further protrusion (23) is configured to engage with the spring element (3) to retain the spring element (3) in the opening (20).

11. The header according to claims 5 and 10, wherein the further protrusion (23) is located on the second end (20b).

12. The header according to claim 10 or 11, wherein the spring element comprises a second end (3b) configured to butt against the further protrusion (23) to retain the spring element (3) in the opening (21).

13. The header according to one of the claims 10 to 12, wherein the further protrusion (23) is an annular protrusion.

14. The header according to one of the claims 10 to 12, wherein the sleeve (2) comprises a plurality of further protrusions (23) located on the second end (20b) of the inner side (20).

15. The header according to one of the preceding claims, wherein the spring element (3) is a bent metal plate and comprises two opposing end portions (31, 32), the respective end portion (31, 32) forming an open ring, and wherein the two end portions (31, 32) are connected by struts (33) being spaced apart in a circumferential direction C1 of the spring element (3).
